# EUROPEAN PATENT SPECIFICATION

(11) **EP 4 153 076 B1**
(45) Date of publication and mention of the grant of the patent: **17.09.2025**
(21) Application number: 21728482.7
(22) Date of filing: 18.05.2021
(51) Int. Cl.: A61B 17/68, A61B 17/72, A61B 17/84, A61B 17/00

(54) **AN ORTHOPAEDIC DEVICE**
ORTHOPÄDISCHE VORRICHTUNG
DISPOSITIF ORTHOPÉDIQUE

(30) Priority: 18.05.2020 US 202063026286 P
(43) Date of publication of application: 29.03.2023
(73) Proprietor: X-Bolt Orthopedics Limited, Dublin 9, D09 AX54 (IE)
(72) Inventor: THORNES, Brian, Dublin, K36EY13 (IE); MCDONALD, Ross, Dublin (IE)
(74) Representative: FRKelly
(86) International application number: PCT/EP2021/063211
(87) International publication number: WO 2021/233955

(56) References cited:
- EP-A1- 3 496 637
- EP-B1- 3 496 637
- US-A1- 2009 005 782
- US-A1- 2015 320 461

## Description

### Field of the Invention

The present invention relates to an orthopaedic device for fixation of bones and a method for the use of the orthopaedic device. The orthopaedic device finds utility for fixation of bones such as fractures of the femur or tibia, although it may be used in any suitable bone.

### Background to the Invention

Bone fixation devices are well known and they find particular utility in the field of orthopaedic surgery, where they are used to fix a bone, which has sustained a fracture, across a fracture site. Generally, the type of fracture determines the type of surgery.

Bone fixation in the presence of osteoporosis is particularly problematic, especially fixation in cancellous (trabecular) bone.

It is an object of the present invention to provide an improved device for bone fixation, optionally femur fixation, which provides a means for reducing the occurrence of screw cut-out, amongst other failure mechanisms, and that will sustain greater weight bearing post-operatively whist the fracture heals. EP 3496637A1 relates to an orthopaedic device comprising a coupling. The orthopaedic device finds utility as a bolt apparatus for fixation of bones such as fractures of the femur, although it may be used in any suitable bone. US 2009/005782A1 relates to a system and methods of using the system to securely fix aligned bone fracture segments in place to promote optimal healing of the fracture. US 2015/320461 A1 relates to a system, including methods and apparatus, for hip fixation with load-controlled dynamization.

### Summary of the Invention

The invention is defined in independent claim 1. Certain optional features of the invention are defined in the dependent claims. According to a first aspect of the present invention there is provided an orthopaedic device comprising:
(a) connecting means comprising at least one pair of threaded portions;
(b) at least two expandable sections, each expandable section displaceable between a contracted position and an expanded position; and
(c) expanding means in operable association with the connecting means and the expandable sections,
wherein rotation of the connecting means causes the expanding means to apply a force to the respective ends of the expandable sections, thereby causing displacement of the expandable sections between the contracted position and the expanded position.

Optionally, the orthopaedic device comprises:
(a) connecting means comprising at least one pair of first and second threaded portions wherein the threads of the first threaded portion are of reverse orientation to the threads of the second threaded portion;
(a) at least two expandable sections, each expandable section displaceable between a contracted position and an expanded position; and
(b) expanding means in operable association with the connecting means and the expandable sections,
wherein rotation of the connecting means causes the expanding means to apply a force to the respective ends of the expandable sections, thereby causing displacement of the expandable sections between the contracted position and the expanded position.

Optionally, the orthopaedic device comprises:
(a) connecting means comprising a pair of first and second threaded portions wherein the threads of the first threaded portion are of reverse orientation to the threads of the second threaded portion;
(b) at least two expandable sections, each expandable section displaceable between a contracted position and an expanded position; and
(c) expanding means in operable association with the connecting means and the expandable sections,
wherein rotation of the connecting means causes the expanding means to apply a force to the respective ends of the expandable sections, thereby causing displacement of the expandable sections between the contracted position and the expanded position.

Preferably, the connecting means is a shaft.

Further preferably, the shaft comprises at least one pair of threaded portions.

Preferably, the orthopaedic device comprises:
(a) a shaft comprising at least one pair of threaded portions wherein the threads of the first threaded portion are of reverse orientation to the threads of the second threaded portion;
(b) at least two expandable sections, each expandable section displaceable between a contracted position and an expanded position; and
(c) expanding means in operable association with the shaft and the expandable sections,
wherein rotation of the shaft causes the expanding means to apply a force to the respective ends of the expandable sections, thereby causing displacement of the expandable sections between the contracted position and the expanded position.

Preferably, the shaft comprises at least one pair of threaded portions comprising a first threaded portion and a second threaded portion.

Optionally, the shaft comprises at least one pair of threaded portions comprising a first threaded portion and a second threaded portion, wherein the threads of the first threaded portion are of reverse orientation to the threads of the second threaded portion
Optionally, the expanding means comprise at least two bodies mountable to the connecting means.

Optionally, the expanding means comprise a first body and a second body, each body mountable to the connecting means.

Optionally, the expanding means comprise at least two bodies threadably mountable to the connecting means.

Optionally, the expanding means comprise a first body threadably mountable and second body threadably mountable to the connecting means.

Optionally, the first body is mountable to the first threaded portion of the connecting means.

Optionally, the second body is mountable to the second threaded portion of the connecting means.

Optionally, the first body is mountable to the first threaded portion and the second body is mountable to the second threaded portion of the connecting means.

Optionally, the first body is threadably mountable to the first threaded portion of the connecting means.

Optionally, the second body is threadably mountable to the second threaded portion of the connecting means.

Optionally, the first body is threadably mountable to the first threaded portion and the second body is threadably mountable to the second threaded portion of the connecting means.

Optionally, the connecting means is a shaft comprising the pair of threaded portions.

Optionally, the connecting means is a shaft comprising the pair of threaded portions, wherein the first body is mountable to the first threaded portion of the connecting means.

Optionally, the connecting means is a shaft comprising the pair of threaded portions, wherein the second body is mountable to the second threaded portion of the connecting means.

Optionally, the connecting means is a shaft comprising the pair of threaded portions, wherein the first body is mountable to the first threaded portion of the connecting means and the second body is mountable to the second threaded portion of the connecting means.

Optionally, the connecting means is a shaft comprising the pair of threaded portions, wherein the first body is mountable to the first threaded portion of the connecting means and the second body is mountable to the second threaded portion of the connecting means to allow for simultaneous movement of the first body and second body in opposite directions.

Optionally, the connecting means is a shaft comprising the pair of threaded portions, wherein the first threaded body is threadably mountable to the first threaded portion of the connecting means. Optionally, the connecting means is a shaft comprising the pair of threaded portions, wherein the second threaded body is threadably mountable to the second threaded portion of the connecting means.

Optionally, the connecting means is a shaft comprising the pair of threaded portions, wherein the first threaded body is threadably mountable to the first threaded portion of the connecting means and the second threaded body is threadably mountable to the second threaded portion of the connecting means.

Optionally, the connecting means is a shaft comprising the pair of threaded portions, wherein the first threaded body is threadably mountable to the first threaded portion of the connecting means and the second threaded body is threadably mountable to the second threaded portion of the connecting means to allow for simultaneous movement of the first threaded body and second threaded body in opposite directions.

Optionally, the connecting means is a shaft comprising the pair of threaded portions, wherein the first threaded body is threadably mountable to the first threaded portion of the connecting means and the second threaded body is threadably mountable to the second threaded portion of the connecting means to allow for simultaneous movement of the first threaded body and second threaded body in opposite directions relative to each other.

Optionally, the first threaded portion and the second threaded portion are each located at or adjacent respective opposing ends of the connecting means. Optionally, the first threaded portion and the second threaded portion are each located at or adjacent respective first and second opposing ends of the connecting means.

Optionally, the first threaded portion and the second threaded portion are located at or adjacent respective opposing ends of the shaft.

Optionally, the first threaded portion and the second threaded portion are each located at or adjacent respective opposing ends of the expandable sections. Optionally, the first threaded portion and the second threaded portion are each located at or adjacent respective first and second opposing ends of the shaft.

Optionally, the orthopaedic device comprises a first expandable section and a second expandable section.

Optionally, the first expandable section comprises a first end and a second end.

Optionally, the second expandable section comprises a third end and a fourth end.

Optionally, the orthopaedic device comprises two expandable sections, the first expandable section comprising a first end and a second end, the second expandable section comprising a third and a fourth end.

Optionally, rotation of the shaft causes the first threadable body to advance along the first threaded portion of the shaft and the second threadable body to advance, in the opposite direction, along the second threaded portion of the shaft, thereby applying mechanical pressure to the first end of the first expandable section and fourth end of the second expandable section, wherein the first end of the first expandable section and fourth end of the second expandable section are bought sequentially into closer proximity relative to one another thereby displacing the first and second expandable sections between the contracted position and the expanded position.

Optionally, the respective ends of the expandable sections refers to each end of the expandable sections where the expanding means applies mechanical pressure, thereby causing displacement of the expandable sections between the contracted position and the expanded position.

Optionally, the first and second expandable sections are arranged contiguously.

Optionally, the first and second expandable sections are reversibly expandable.

Optionally, the first and second expandable sections are arranged contiguously and/or are reversibly expandable.

Optionally, the first and second expandable sections are arranged contiguously and are reversibly expandable.

Further optionally, the first and second expandable sections are reversibly expandable under mechanical pressure.

Optionally, the connecting means further comprises a further pair of threaded portions.

Optionally, the connecting means comprise at least two pairs of threaded portions.

Optionally, the connecting means comprise two pairs of threaded portions.

Optionally, the connecting means comprise four threaded portions comprising a first threaded portion, a second threaded portion, a third threaded portion and a fourth threaded portion. Optionally, the connecting means comprise four threaded portions comprising a first threaded portion, a second threaded portion, a third threaded portion and a fourth threaded portion.

Optionally, the connecting means is a shaft which comprises four threaded portions comprising a first threaded portion, a second threaded portion, a third threaded portion and a fourth threaded portion.

Optionally, the connecting means comprise two pairs of threaded portions, the first pair comprising the first and second threaded portions and the second pair comprising a third threaded portion and a fourth threaded portion.

Optionally, the shaft comprises two pairs of threaded portions, the first pair comprising the first and second threaded portions and the second pair comprising a third threaded portion and a fourth threaded portion.

Optionally, the connecting means comprise four threaded portions comprising a first threaded portion, a second threaded portion, a third threaded portion and a fourth threaded portion, wherein the first threaded portion, the second threaded portion, the third threaded portion and the fourth threaded portion are arranged sequentially along the length of the shaft in the order of the first threaded portion, the third threaded portion, the fourth threaded portion and the second threaded portion.

Optionally, the connecting means is a shaft which comprises four threaded portions comprising a first threaded portion, a second threaded portion, a third threaded portion and a fourth threaded portion, wherein the first threaded portion, the second threaded portion, the third threaded portion and the fourth threaded portion are arranged sequentially along the length of the shaft in the order of the first threaded portion, the third threaded portion, the fourth threaded portion and the second threaded portion.

Optionally, the threads of the first threaded portion are in reverse orientation to the threads of the second threaded portion.

Optionally, the threads of the third threaded portion are in reverse orientation to the threads of the fourth threaded portion.

Optionally, the threads of the first threaded portion are in reverse orientation to the threads of the third threaded portion.

Optionally, the threads of the second threaded portion are in reverse orientation to the threads of the fourth threaded portion.

Optionally, the expanding means comprise at least four bodies, each body mountable to the connecting means.

Optionally, the expanding means comprise four bodies, each body mountable to the connecting means.

Optionally, the expanding means comprise four bodies, each body mountable to the shaft. Optionally, the expanding means comprise four bodies, each body threadably mountable to the connecting means.

Optionally, the expanding means comprise four bodies, each body threadably mountable to the shaft.

Optionally, the expanding means comprise four bodies, each body mountable to the threaded portions of the connecting means.

Optionally, the expanding means comprise four bodies, each body mountable to the threaded portions of the shaft.

Optionally, the expanding connecting means comprise four bodies, each body threadably mountable to the threaded portions of the connecting means.

Optionally, the expanding means comprise four bodies, each body threadably mountable to the threaded portions of the shaft.

Optionally, the expanding means comprise a first body, a second body and third body and a fourth body.

Optionally, the expanding means comprise a first threadable body, a second threadable body, a third threadable body and a fourth threadable body.

Optionally, the first body is engagable with the first threaded portion of the connecting means.

Optionally, the first body is engagable with the first threaded portion of the shaft.

Optionally, the second body is engagable with the second threaded portion of the connecting means.

Optionally, the second body is engagable with the second threaded portion of the shaft.

Optionally, the third body is engagable with the third threaded portion of the connecting means.

Optionally, the third body is engagable with the third threaded portion of the shaft.

Optionally, the fourth body is engagable with the fourth threaded portion of the connecting means.

Optionally, the fourth body is engagable with the fourth threaded portion of the shaft.

Optionally, the first threadable body is engagable with the first threaded portion of the connecting means.

Optionally, the first threadable body is engagable with the first threaded portion of the shaft.

Optionally, the second threadable body is engagable with the second threaded portion of the connecting means.

Optionally, the second threadable body is engagable with the second threaded portion of the shaft.

Optionally, the third threadable body is engagable with the third threaded portion of the connecting means.

Optionally, the third threadable body is engagable with the third threaded portion of the shaft.

Optionally, the fourth threadable body is engagable with the fourth threaded portion of the connecting means.

Optionally, the fourth threadable body is engagable with the fourth threaded portion of the shaft.

Optionally, the connecting means comprise first and second pairs of threaded portions, wherein the first body engages with the first threaded portion, the second body engages with the second threaded portion, the third body engages with the third threaded portion and the fourth body engages with the fourth threaded portion.

Optionally, the connecting means comprise first and second pairs of threaded portions, wherein the first threadable body engages with the first threaded portion, the second threadable body engages with the second threaded portion, the third threadable body engages with the third threaded portion and the fourth threadable body engages with the fourth threaded portion.

Optionally, the connecting means is a shaft comprising first and second pairs of threaded portions, wherein the first body engages with the first threaded portion, the second body engages with the second threaded portion, the third body engages with the third threaded portion and the fourth body engages with the fourth threaded portion.

Optionally, the connecting means is a shaft comprising first and second pairs of threaded portions, wherein the first threadable body engages with the first threaded portion, the second threadable body engages with the second threaded portion, the third threadbare body engages with the third threaded portion and the fourth threadable body engages with the fourth threaded portion.

Optionally, the connecting means comprise first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and second body in opposite directions relative to each other; and the third body and fourth body in opposite directions relative to each other.

Optionally, the connecting means comprise first and second pairs of threaded portions, wherein the first threaded body engages with the first threadable portion, the second threaded body engages with the second threadable portion, the third threaded body engages with the third threadable portion and the fourth threaded body engages with the fourth threaded portion to allow for simultaneous movement of the first threadable body and second threadable body in opposite directions relative to each other; and the third threadable body and fourth threadable body in opposite directions relative to each other.

Optionally, the connecting means is a shaft comprising first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and second body in opposite directions relative to each other; and the third body and fourth body in opposite directions relative to each other.

Optionally, the connecting means is a shaft comprising first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and second threadable body in opposite directions relative to each other; and the third threadable body and fourth threadable body in opposite directions relative to each other.

Optionally, the connecting means comprise first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and third body in opposite directions relative to each other; and second body and fourth body in opposite directions relative to each other.

Optionally, the connecting means comprise first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and third threadable body in opposite directions relative to each other; and second threadable body and fourth threadable body in opposite directions relative to each other.

Optionally, the connecting means is a shaft comprising first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and third body in opposite directions relative to each other; and the second body and the fourth body in opposite directions relative to each other.

Optionally, the connecting means is a shaft comprising first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and the third threadable body in opposite directions relative to each other; and the second threadable body and the fourth threadable body in opposite directions relative to each other.

Optionally, the orthopaedic device comprises a first expandable section and a second expandable section.

Optionally, the first expandable section comprises a first end and a second end.

Optionally, the second expandable section comprises a third end and a fourth end.

Optionally, the orthopaedic device comprises two expandable sections, the first expandable section comprising a first end and a second end, the second expandable section comprising a third and a fourth end.

Optionally, the connecting means comprise first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and the second body in opposite directions relative to each other; and the third body and the fourth body in opposite directions relative to each other to displace the first and second expandable sections between the contracted position and the expanded position by applying force to the first and second end of the first expandable section and the third and fourth end of the second expandable section.

Optionally, the connecting means comprise first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and the second threadable body in opposite directions relative to each other; and the third threadable body and the fourth threadable body in opposite directions relative to each other to displace the first and second expandable sections between the contracted position and the expanded position by applying force to the first and second end of the first expandable section and the third and fourth end of the second expandable section.

Optionally, the connecting means is a shaft comprising first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and second body in opposite directions relative to each other; and the third body and fourth body in opposite directions relative to each other to displace the first and second expandable sections between the contracted position and the expanded position by applying force to the first and second end of the first expandable section and the third and fourth end of the second expandable section.

Optionally, the connecting means is a shaft comprising first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and the second threadable body in opposite directions relative to each other; and the third threadable body and the fourth threadable body in opposite directions relative to each other to displace the first and second expandable sections between the contracted position and the expanded position by applying force to the first and second end of the first expandable section and the third and fourth end of the second expandable section.

Optionally, the connecting means comprise first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and the third body in opposite directions relative to each other; and the second body and the fourth body in opposite directions relative to each other to displace the first and second expandable sections between the contracted position and the expanded position by applying force to the first and second end of the first expandable section and the third and fourth end of the second expandable section.

Optionally, the connecting means comprise first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and the third threadable body in opposite directions relative to each other; and the second threadable body and the fourth threadable body in opposite directions relative to each other to displace the first and second expandable sections between the contracted position and the expanded position by applying force to the first and second end of the first expandable section and the third and fourth end of the second expandable section.

Optionally, the connecting means is a shaft comprising first and second pairs of threaded portions, wherein the first body engages with the first threaded portion, the second body engages with the second threaded portion, the third body engages with the third threaded portion and the fourth body engages with the fourth threaded portion to allow for simultaneous movement of the first body and third body in opposite directions relative to each other; and the second body and the fourth body in opposite directions relative to each other to displace the first and second expandable sections between the contracted position and the expanded position by applying force to the first and second end of the first expandable section and the third and fourth end of the second expandable section.

Optionally, the connecting means is a shaft comprising first and second pairs of threaded portions, wherein the first threadable body engages with the first threaded portion, the second threadable body engages with the second threaded portion, the third threadable body engages with the third threaded portion and the fourth threadable body engages with the fourth threaded portion to allow for simultaneous movement of the first threadable body and third threadable body in opposite directions relative to each other; and second threadable body and fourth threadable body in opposite directions relative to each other to displace the first and second expandable sections between the contracted position and the expanded position by applying force to the first and second end of the first expandable section and the third and fourth end of the second expandable section.

Optionally, each or any of the bodies are arranged for displacement in response to rotation of the connecting means to apply a force to the first and second end of the first expandable section and the third and fourth end of the second expandable section.

Optionally, each or any of the bodies are arranged for displacement in response to rotation of the shaft to apply a force to the first and second end of the first expandable section and the third and fourth end of the second expandable section.

Optionally, each or any of the threadable bodies are arranged for displacement in response to rotation of the connecting means to apply a force to the first and second end of the first expandable section and the third and fourth end of the second expandable section.

Optionally, each or any of the threadable bodies are arranged for displacement in response to rotation of the shaft to apply a force to the first and second end of the first expandable section and the third and fourth end of the second expandable section.

Optionally, at least one end of the connecting means is dimensioned and arranged to provide means for rotating the threaded shaft.

Optionally, at least one end of the shaft is dimensioned and arranged to provide means for rotating the threaded shaft.

Optionally, at least one end of the connecting means is dimensioned and arranged to provide means for delivering torque to the connecting means.

Optionally, at least one end of the shaft is dimensioned and arranged to provide means for delivering torque to the shaft.

Optionally, at least one end of the connecting means is dimensioned and arranged to receive a torque delivery device such as a screwdriver, hex key, or similar device.

Optionally, at least one end of the shaft is dimensioned and arranged to receive a torque delivery device such as a screwdriver, hex key, or similar device.

Optionally, the first and second expandable sections are arranged contiguously.

Optionally, the first and second expandable sections are reversibly expandable.

Optionally, the first and second expandable sections are arranged contiguously and/or are reversibly expandable.

Optionally, the first and second expandable sections are arranged contiguously and are reversibly expandable.

Further optionally, the first and second expandable sections are reversibly expandable under mechanical pressure.

Optionally, the first and second expandable sections are collapsible along their longitudinal axis.

Further optionally, the first and second expandable sections are radially inwardly collapsible.

Optionally, the first and second expandable sections each comprise a deformable arm.

Optionally, each or any of the points of folding comprises a point of weakness, a hinge mechanism, or any such mechanism that will facilitate the folding of the deformable arm at a desired location.

Optionally, the orthopaedic device further comprises limiting means.

Optionally, the limiting means limit displacement of at least one expandable section between the contracted position and the expanded position.

Optionally, the limiting means limit displacement of at least one expandable section between the contracted position and the expanded position by preventing the respective ends of the at least one expandable section from moving sequentially into closer proximity relative to one another.

Optionally, the shaft comprises the limiting means.

Optionally, at least one threadable body comprises the limiting means.

Optionally, at least one expandable section comprises the limiting means.

Optionally, at least one end of the at least one expandable section comprises the limiting means.

Optionally, the limiting means are coaxial with the longitudinal axis of the shaft.

Optionally or additionally, the limiting means are colinear.

Optionally, at least one expandable section comprises limiting means which are colinear with the longitudinal axis of the shaft.

Optionally, at least one expandable section comprises limiting means which are arranged non-contiguously.

Optionally, at least one expandable section comprises limiting means which are colinear with the longitudinal axis of the shaft or are arranged non-contiguously.

Optionally, at least one expandable section comprises limiting means which are colinear with the longitudinal axis of the shaft and are arranged non-contiguously.

Optionally, at least one expandable section comprises limiting means which are colinear with the longitudinal axis of the shaft and are arranged non-contiguously thereby forming a space between the limiting means.

Optionally, the limiting means limits displacement of the at least one expandable section by preventing the first end of the first expandable section and/or the fourth end of the second expandable section from moving sequentially into closer proximity relative to one another.

Optionally, the limiting means limits displacement of the first expandable section by preventing the first end of the first expandable section and/or the second end of the first expandable section from moving sequentially into closer proximity relative to one another.

Optionally or additionally, the limiting means limits displacement of the second expandable section by preventing the third end of the second expandable section and/or the fourth end of the second expandable section from moving sequentially into closer proximity relative to one another.

Optionally, the limiting means comprise at least one stop.

Optionally, the limiting means comprise two stops.

Optionally, the limiting means comprise two collinear stops.

Optionally, the limiting means comprise two non-contiguously arranged stops.

Optionally, the limiting means comprise two colinear non-contiguously arranged stops.

Optionally, the limiting means are arranged to form a stop.

Optionally, the stop limits displacement of the at least one expandable section between the contracted position and the expanded position by preventing the respective ends of the at least one expandable section from moving sequentially into closer proximity relative to one another.

Optionally, the stop limits displacement of the at least one expandable section by preventing the first end of the first expandable section and/or the fourth end of the second expandable section from moving sequentially into closer proximity relative to one another.

Optionally, the stop limits displacement of the first expandable section by preventing the first end of the first expandable section and/or the second end of the first expandable section from moving sequentially into closer proximity relative to one another.

Optionally or additionally, the stop limits displacement of the second expandable section by preventing the third end of the second expandable section and/or the fourth end of the second expandable section from moving sequentially into closer proximity relative to one another.

Optionally, rotation of the shaft causes the first threadable body to advance along the first threaded portion of the shaft and the second threadable body to advance, in the opposite direction, along the second threaded portion of the shaft, thereby applying mechanical pressure to the first end of the first expandable section and fourth end of the second expandable section, wherein the limiting means limits the displacement of the at least one expandable section by preventing the first end of the first expandable section and/or the fourth end of the second expandable section from moving sequentially into closer proximity relative to one another, thereby limiting the displacement of the at least one expandable section.

Optionally, rotation of the shaft causes the first threadable body to advance along the first threaded portion of the shaft and the second threadable body to advance, in the opposite direction, along the second threaded portion of the shaft, thereby applying mechanical pressure to the first end of the first expandable section and fourth end of the second expandable section, wherein the stop limits displacement of the at least one expandable section by preventing the first end of the first expandable section and/or the fourth end of the second expandable section from moving sequentially into closer proximity relative to one another, thereby limiting the displacement of the at least one expandable section.

Optionally, the limiting means is at least one strut.

Optionally, the limiting means is a first strut or a second strut.

Optionally, the limiting means is a first strut and a second strut.

Optionally, the first strut and the second strut limits displacement of the at least one expandable section by preventing the respective ends of the expandable sections from moving sequentially into closer proximity relative to one another.

Optionally, at least one expandable section comprises the first strut and the second strut.

Optionally, at least one expandable section comprises the first strut and the second strut which are colinear with the longitudinal axis of the shaft.

Optionally, at least one expandable section comprises the first strut and the second strut which are arranged non-contiguously.

Optionally, at least one expandable section comprises the first strut and the second strut, which are colinear with the longitudinal axis of the shaft or are arranged non-contiguously.

Optionally, at least one expandable section comprises the first strut and the second strut, which are colinear with the longitudinal axis of the shaft and are arranged non-contiguously.

Optionally, at least one expandable section comprises the first strut and the second strut, which are colinear with the longitudinal axis of the shaft and are arranged non-contiguously thereby forming a space between the first strut and the second strut.

Optionally, at least one expandable section comprises a first strut and a second strut, which are colinear with the longitudinal axis of the shaft and are arranged non-contiguously, wherein rotation of the shaft causes the first threadable body to advance along the first threaded portion of the shaft and the second threadable body to advance, in the opposite direction, along the second threaded portion of the shaft, thereby applying mechanical pressure to the first end of the first expandable section and fourth end of the second expandable section, wherein the first end of the first expandable section and fourth end of the second expandable section are bought sequentially into closer proximity relative to one another, which closes the space between the first strut and second strut until the first strut and a second strut meet, thereby limiting displacement of the at least one expandable section.

Preferably, there is provided an orthopaedic device comprising:
(a) connecting means comprising at least one pair of threaded portions;
(b) at least one expandable section, each expandable section displaceable between a contracted position and an expanded position,
(c) expanding means in operable association with connecting means and the expandable sections, wherein rotation of connecting means causes the expanding means to apply a force to the respective ends of the expandable sections, thereby causing displacement of the expandable sections between the contracted position and the expanded position; and
(d) limiting means for limiting displacement of the at least one expandable section by preventing the respective ends of the expandable sections from moving sequentially into closer proximity relative to one another.

Optionally, there is provided an orthopaedic device comprising:
(a) a shaft comprising at one pair of threaded portions;
(b) at least one expandable section, each expandable section displaceable between a contracted position and an expanded position,
(c) expanding means in operable association with the shaft and the expandable sections, wherein rotation of the shaft causes the expanding means to apply a force to the respective ends of the expandable sections, thereby causing displacement of the expandable sections between the contracted position and the expanded position; and
(d) limiting means for limiting displacement of the at least one expandable section by preventing the respective ends of the expandable sections from moving sequentially into closer proximity relative to one another.

Optionally, there provided an orthopaedic device comprising:
(a) a shaft comprising at two pairs of threaded portions;
(b) at least one expandable section, each expandable section displaceable between a contracted position and an expanded position,
(c) expanding means in operable association with the shaft and the expandable sections, wherein rotation of the shaft causes the expanding means to apply a force to the respective ends of the expandable sections, thereby causing displacement of the expandable sections between the contracted position and the expanded position; and
(d) limiting means for limiting displacement of the at least one expandable section by preventing the respective ends of the expandable sections from moving sequentially into closer proximity relative to one another.

According to a second aspect of the present invention, there is provided an orthopaedic system comprising:
(a) an orthopaedic device according to a first aspect of the present invention; and
(b) an orthopaedic implant.

Optionally, at least part of the orthopaedic device has a substantially non-circular cross-section.

Optionally, at least part of the outer surface of the orthopaedic device has a substantially non-circular shape.

Optionally, the orthopaedic implant comprises at least one aperture.

Optionally, the orthopaedic implant comprises at least one non-parallel aperture.

Optionally, the orthopaedic implant comprises at least one transverse aperture.

Optionally, the orthopaedic implant comprises at least one transverse aperture which bisects the longitudinal axis relative to the orthopaedic implant.

Optionally, the orthopaedic implant is adapted to receive the orthopaedic device according to a first aspect of the present invention.

Optionally, the transverse aperture is substantially circular in shape.

Alternatively, the transverse aperture is substantially non-circular in shape.

Optionally, the transverse aperture comprises a first part and a second part.

Optionally, the transverse aperture comprises a first part and a second part, wherein the first part is circular.

Optionally, the transverse aperture comprises a first part and a second part, wherein the second part is non-circular.

Optionally, the transverse aperture comprises a first part and a second part, wherein the first part is circular and the second part is non-circular.

Optionally, the orthopaedic device has a substantially circular cross-section.

Preferably, the orthopaedic device has a substantially non-circular cross-section.

Optionally, the orthopaedic device has a substantially non-circular cross-section comprising at least one linear section.

Optionally, the orthopaedic device has a substantially non-circular cross-section comprising at least two linear sections.

Optionally, the orthopaedic device has a substantially non-circular cross-section comprising at least one curved section.

Optionally, the orthopaedic device has a substantially non-circular cross-section comprising at least two curved sections.

Preferably, the orthopaedic device has a substantially non-circular cross-section comprising at least one linear section and at least one curved section.

Optionally, the orthopaedic device has a substantially non-circular cross-section comprising at least two linear sections and at least two curved sections.

Optionally, the outer surface the orthopaedic device comprises at least one planar section.

Optionally, the outer surface the orthopaedic device comprises at least two planar sections.

Optionally, the outer surface the orthopaedic device comprises at least one curved section.

Optionally, the outer surface the orthopaedic device comprises at least two curved sections.

Preferably, the outer surface the orthopaedic device comprises at least one planar section and at least one curved section.

Optionally, the outer surface the orthopaedic device comprises at least two planar sections and at least two curved sections.

Optionally, the orthopaedic device has a substantially non-circular cross-section comprising at least one linear section and at least one curved section, wherein the least one linear cross-section section corresponds to the at least one planar section on the outer surface of the orthopaedic device, and the at least one curved cross-section corresponds to the at least one curved section on the outer surface of the orthopaedic device.

Optionally, the orthopaedic device has a substantially non-circular cross-section comprising at least two linear sections and at least two curved sections, wherein the least two linear cross-section sections corresponds to the at least two planar sections on the outer surface of the orthopaedic device, and the at least two curved cross-sections corresponds to the at least two curved sections on the outer surface of the orthopaedic device.

Optionally, the at least one linear cross-section of the orthopaedic device and the at least one curved cross-sections of the orthopaedic device are diametrically opposed.

Optionally, the at least one planar section on the outer surface of the orthopaedic device and the at least one curved section on the outer surface of the orthopaedic device are diametrically opposed.

Optionally, the at least two linear cross-sections of the orthopaedic device and the at least two curved cross-sections of the orthopaedic device are diametrically opposed.

Optionally, the at least two planar sections on the outer surface of the orthopaedic device and the at least two curved sections on the outer surface of the orthopaedic device are diametrically opposed.

Optionally, the transverse aperture comprises a first part and a second part, wherein the first part is circular to receive the curved outer section of the orthopaedic device.

Optionally, the transverse aperture comprises a first part and a second part, wherein the second part is non-circular to receive the flat outer section of the orthopaedic device.

Optionally, the transverse aperture comprises a first part and a second part, wherein the first part is circular to receive the curved outer section of the orthopaedic device, and the second part is non-circular to receive the flat outer section of the orthopaedic device.

Optionally, the transverse aperture has a keyhole shape.

Preferably, the orthopaedic implant is an intramedullary nail.

Optionally, the intramedullary nail comprises at least one aperture.

Optionally, the intramedullary nail comprises at least one non-parallel aperture.

Optionally, the intramedullary nail comprises at least one transverse aperture.

Optionally, the intramedullary nail comprises at least one transverse aperture which bisects the longitudinal axis relative to the intramedullary nail.

Optionally, the intramedullary nail is adapted to receive the orthopaedic device.

Optionally, the transverse aperture is substantially circular in shape.

Alternatively, the transverse aperture is substantially non-circular in shape.

Optionally, the transverse aperture comprises a first part and a second part.

Optionally, the transverse aperture comprises a first part and a second part, wherein the first part is circular.

Optionally, the transverse aperture comprises a first part and a second part, wherein the second part is non-circular.

Optionally, the transverse aperture comprises a first part and a second part, wherein the first part is circular and the second part is non-circular.

Optionally, the orthopaedic system further comprises a locking means.

Optionally, the locking means is threadably mountable to the orthopaedic implant.

Optionally, the locking means is threadably mountable to the proximal end of the orthopaedic implant.

Alternatively, the locking means is threadably mountable to the distal end of the orthopaedic implant.

Optionally, the orthopaedic implant further comprises a longitudinal aperture.

Optionally, the orthopaedic implant further comprises a longitudinal aperture which corresponds to the proximal end of the orthopaedic implant.

Alternatively, the orthopaedic implant further comprises a longitudinal aperture which corresponds to the distal end of the orthopaedic implant.

Optionally, the orthopaedic implant further comprises a longitudinal aperture coaxial with the longitudinal axis.

Optionally, the orthopaedic implant further comprises a longitudinal aperture coaxial with the longitudinal axis, wherein the longitudinal aperture comprises an inner threaded surface.

Optionally, the orthopaedic implant further comprises a longitudinal aperture coaxial with the longitudinal axis, wherein the longitudinal aperture comprises an inner threaded surface and is in mechanical communication with the transverse aperture.

Preferably, the locking means comprises a set-screw.

Optionally, the set-screw is threadably mountable to the longitudinal aperture of the orthopaedic implant, wherein rotation of the set-screw causes the set-screw to engage with the orthopaedic implant.

Optionally, the set-screw is threadably mountable to the longitudinal aperture of the orthopaedic implant, wherein rotation of the set-screw causes the set-screw to engage with the second part of the orthopaedic implant.

Optionally, the transverse aperture comprising the first part for receiving the curved outer section of the orthopaedic device and the second part for receiving the planar outer section of the orthopaedic device is in mechanical communication with the longitudinal aperture of the orthopaedic implant, wherein the set-screw is threadably mountable to the longitudinal aperture of the orthopaedic implant and rotation of the set-screw causes the set-screw to engage with the second part of the orthopaedic implant.

Optionally, the transverse aperture comprising the first part for receiving the curved outer section of the orthopaedic device and the second part for receiving the planar outer section of the orthopaedic device is in mechanical communication with the longitudinal aperture of the orthopaedic implant, wherein the set-screw is threadably mountable to the longitudinal aperture of the orthopaedic implant and rotation of the set-screw causes the set-screw to engage with the second part of the orthopaedic implant, thus preventing further rotation of the orthopaedic device in the orthopaedic implant.

Optionally, the transverse aperture comprising the first part for receiving the curved outer section of the orthopaedic device and the second part for receiving the planar outer section of the orthopaedic device is in mechanical communication with the longitudinal aperture of the orthopaedic implant, wherein the set-screw is threadably mountable to the longitudinal aperture of the orthopaedic implant and rotation of the set-screw causes the set-screw to displace the orthopaedic device from the first part of the transverse aperture to the second part of the transverse aperture.

Optionally, the transverse aperture comprising the first part for receiving the curved outer section of the orthopaedic device and the second part for receiving the planar outer section of the orthopaedic device is in mechanical communication with the longitudinal aperture of the orthopaedic implant, wherein the set-screw is threadably mountable to the longitudinal aperture of the orthopaedic implant and rotation of the set-screw causes the set-screw to displace the orthopaedic device from the first part of the transverse aperture to the second part of the transverse aperture by driving the orthopaedic device along the longitudinal axis of the orthopaedic implant.

Optionally, the transverse aperture comprising the first part for receiving the curved outer section of the orthopaedic device and the second part for receiving the planar outer section of the orthopaedic device is in mechanical communication with the longitudinal aperture of the orthopaedic implant, wherein the set-screw is threadably mountable to the longitudinal aperture of the orthopaedic implant and rotation of the set-screw causes the set-screw to displace the orthopaedic device from the first part of the transverse aperture to the second part of the transverse aperture by driving the orthopaedic device along the longitudinal axis of the orthopaedic implant such that further rotation of the orthopaedic device in the orthopaedic implant is prevented.

Optionally, the set-screw is threadably mountable to the longitudinal aperture of the intramedullary nail wherein rotation of the set-screw causes the set-screw to engage with the intramedullary nail. Optionally, the set-screw is threadably mountable to the longitudinal aperture of the intramedullary nail, wherein rotation of the set-screw causes the set-screw to engage with the second part of the intramedullary nail.

Optionally, the transverse aperture comprising the first part for receiving the curved outer section of the orthopaedic device and the second part for receiving the planar outer section of the orthopaedic device is in mechanical communication with the longitudinal aperture of the intramedullary nail, wherein the set-screw is threadably mountable to the longitudinal aperture of the intramedullary nail and rotation of the set-screw causes the set-screw to engage with the second part of the intramedullary nail.

Optionally, the transverse aperture comprising the first part for receiving the curved outer section of the orthopaedic device and the second part for receiving the planar outer section of the orthopaedic device is in mechanical communication with the longitudinal aperture of the intramedullary nail, wherein the set-screw is threadably mountable to the longitudinal aperture of the intramedullary nail and rotation of the set-screw causes the set-screw to engage with the second part of the orthopaedic implant, thus preventing further rotation of the orthopaedic device in the intramedullary nail.

Optionally, the transverse aperture comprising the first part for receiving the curved outer section of the orthopaedic device and the second part for receiving the planar outer section of the orthopaedic device is in mechanical communication with the longitudinal aperture of the intramedullary nail, wherein the set-screw is threadably mountable to the longitudinal aperture of the intramedullary nail and rotation of the set-screw causes the set-screw to displace the orthopaedic device from the first part of the transverse aperture to the second part of the transverse aperture.

Optionally, the transverse aperture comprising the first part for receiving the curved outer section of the orthopaedic device and the second part for receiving the planar outer section of the orthopaedic device is in mechanical communication with the longitudinal aperture of the intramedullary nail, wherein the set-screw is threadably mountable to the longitudinal aperture of the intramedullary nail and rotation of the set-screw causes the set-screw to displace the orthopaedic device from the first part of the transverse aperture to the second part of the transverse aperture by driving the orthopaedic device along the longitudinal axis of the intramedullary nail.

Optionally, the transverse aperture comprising the first part for receiving the curved outer section of the orthopaedic device and the second part for receiving the planar outer section of the orthopaedic device is in mechanical communication with the longitudinal aperture of the intramedullary nail, wherein the set-screw is threadably mountable to the longitudinal aperture of the intramedullary nail and rotation of the set-screw causes the set-screw to displace the orthopaedic device from the first part of the transverse aperture to the second part of the transverse aperture by driving the orthopaedic device along the longitudinal axis of the orthopaedic implant such that further rotation of the orthopaedic device in the intramedullary nail is prevented.

Optionally, the transverse aperture comprising the first part for receiving the curved outer section of the orthopaedic device and the second part for receiving the planar outer section of the orthopaedic device is in mechanical communication with the longitudinal aperture of the orthopaedic implant, wherein the locking means is threadably mountable to the longitudinal aperture of the orthopaedic implant and rotation of the set-screw causes the locking means to displace the orthopaedic device from the first part of the transverse aperture to the second part of the transverse aperture by driving the orthopaedic device along the longitudinal axis of the orthopaedic implant such that further rotation of the orthopaedic device in the orthopaedic implant is prevented.

Optionally, the orthopaedic device fixes the femur.

Optionally, the orthopaedic device fixes at the distal end of the femur.

Optionally, the orthopaedic device fixes the tibia.

Optionally, the orthopaedic device fixes at the proximal end of the femur.

Optionally, the orthopaedic device fixes any suitable bone.

According to a further aspect of the present invention, there is provided a method for fixation of bones, the method comprising the steps of reducing the fracture; providing a channel across the fracture; inserting an orthopaedic device according to a first aspect of the invention in the channel; and fixing the orthopaedic device in the channel.

Optionally, the fixing step comprises displacing the expandable sections toward the expanded position by applying force to the respective ends of the expandable sections, such that each of the respective ends of the expandable sections are advanced toward the opposing respective end.

Additionally, there is provided a method for fixation of bones, the method comprising the steps of reducing the fracture; providing a channel across the fracture; inserting an orthopaedic implant in the channel and an orthopaedic device according to a first aspect of the present invention into an aperture on the orthopaedic implant; and fixing the orthopaedic device in the channel.

Additionally, there is provided a method for fixation of bones, the method comprising the steps of reducing the fracture; providing a channel across the fracture; inserting an intramedullary nail in the channel and an orthopaedic device according to a first aspect of the present invention into an aperture on the intramedullary nail; and fixing the orthopaedic device in the channel.

Optionally, the fixing step comprises displacing the expandable sections toward the expanded position by applying force to the respective ends of the expandable sections, such that each of the respective ends of the expandable sections are advanced toward the opposing respective end.

### Brief Description of the Drawings

Embodiments of the invention will now be described with reference to the accompanying drawings, in which:
**Figure 1** is a perspective view of an orthopaedic device in an assembled contracted position according to a first aspect of the present invention;
**Figure 2** is an exploded perspective view of the orthopaedic device of Figure 1 according to the first embodiment of the present invention;
**Figure 3** is an exploded perspective view an orthopaedic device according to the second embodiment of the first aspect of the present invention;
**Figure 4** is a perspective view the orthopaedic device of Figure 1 in an assembled expanded position;
**Figure 5** is a plan view of an orthopaedic device with a set screw;
**Figure 6** is a perspective view the orthopaedic device of Figure 5 in an assembled contracted position;
**Figure 7** is a perspective view the orthopaedic device of Figure 5 in an assembled expanded position; and
**Figure 8** is a perspective view of the orthopaedic device further comprising limiting means.

### Detailed Description of the Invention

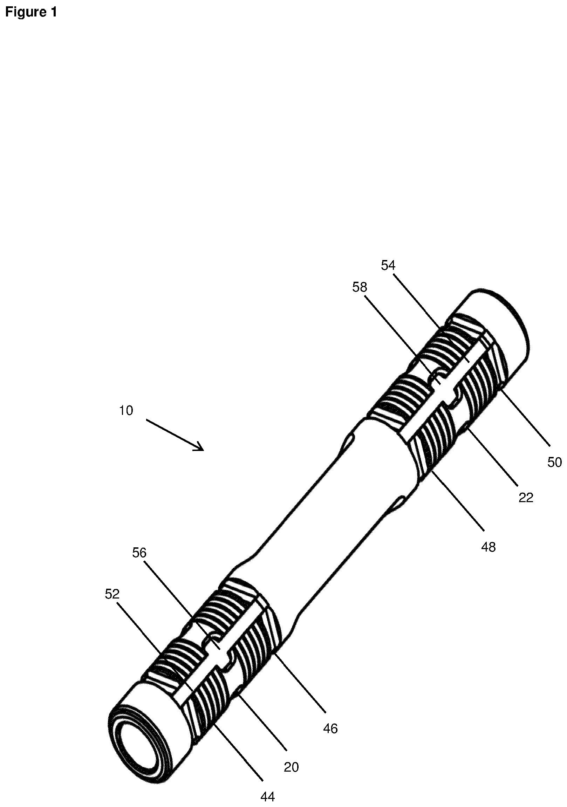

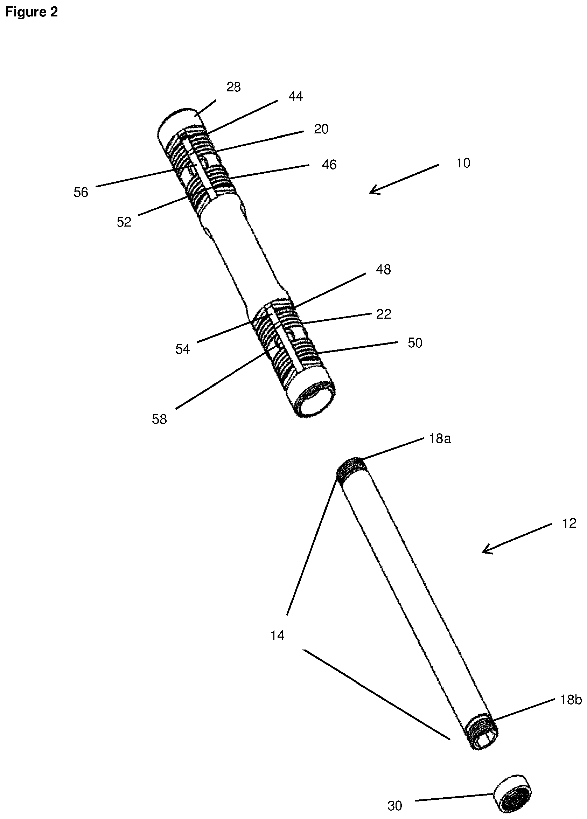

Referring to Figure 1 and Figure 2 of the drawings, there is provided an orthopaedic device 10 according to a first embodiment of the present invention. The orthopaedic device 10 comprises connecting means, which in this embodiment is a shaft 12; a first pair of threaded portions 14 comprising first 18a and second 18b threaded portions wherein the threads of the first threaded portion 18a are of reverse orientation to the threads of the second threaded portion 18b; two expandable sections - a first expandable section 20 and a second expandable section 22 - the first 20 and second 22 expandable sections displaceable between a contracted position and an expanded position; and expanding means, which in this embodiment is a first threadable body 28 and second threadable body 30, which are in operable association with the shaft 12 and the expandable sections 20, 22.

The shaft 12 allows reciprocal movement of the threadably mountable bodies 28, 30 relative to the shaft 12. The threaded portions 18a, 18b on the shaft 12 are each helically threaded portions. The threads of the first helical screw threaded portion 18a are in reverse orientation to the threads of the second helical screw threaded portion 18b. The two helical screw threaded portions 18a, 18b are provided adjacent each terminal end of the shaft 12.

The shaft 12 is generally cylindrical in shape and each terminal end of the shaft 12 comprises a recess to actuate the shaft 12. One end of the shaft 12 is dimensioned and arranged to provide means for actuating the shaft 12, for example by providing means for delivering torque to the shaft. The terminal ends of the shaft 12 can be dimensioned and arranged to receive a torque delivery device such as a screwdriver, hex key, or similar device. A hexagonal socket (not shown) can be provided at the terminal ends of the shaft 12, and can be shaped and adapted to receive a hex key or similar torque delivery device.

In an embodiment, the threaded portions 18a, 18b are spaced apart at the terminal ends of the shaft 12. In use, the orthopaedic device 10 comprising the shaft 12 crosses an intramedullary nail 60, so that the first threaded portion 18a is one side of the intramedullary nail 60 and the second threaded portion 18b is on the other side of the intramedullary nail 60.

The threadably mountable bodies 28, 30 are annular in shape having an internal threaded surface and are open at both ends. The inner shape of the threadably mountable bodies 28, 30 are shaped and dimensioned to engage with the threaded shaft of an orthopaedic device.

A helical screw thread can be provided on the inner surface of each threadably mountable body 28, 30. The internal diameter of each threadably mountable body 28, 30 is generally of similar length to the external diameter of the shaft 12, whereby the screw thread of each threadably mountable body 28, 30 can engage with the respective screw threaded portions 18a, 18b of the shaft 12.

Each expandable section 20, 22 comprise a generally cylindrically shaped tube, which is open at both ends. The first expandable section 20 and the second expandable section 22 are located coaxially. The first expandable section 20 comprises deformable arms 52, the second expandable section 22 also comprises deformable arms 54, such that the expandable sections 20, 22 comprises a plurality of arms 52, 54 that extend from the longitudinal axis of the orthopaedic device 10 under mechanical pressure. A point of weakness 56 is provided at the first end 44 and second end 46 of the first expandable section of the deformable arm 52, and generally at the centre point of the length of the deformable arm 52. A point of weakness 58 is also provided at the third end 48 and fourth end 50 of the second expandable section of the deformable arm 54, and generally at the centre point of the length of the deformable arm 54. Each deformable arm 52, 54 have at least one point of folding 56, 58 along each deformable arm 52, 54. Each of the points of folding 56, 58 comprises a point of weakness, a hinge mechanism, or any such mechanism that will facilitate the folding of the deformable arm at a desired location.

The first 20 and second 22 expandable sections can be reversibly expandable. The first 20 and second 22 expandable sections are collapsible along their longitudinal axis, such that the first 20 and second 22 expandable sections are radially inwardly collapsible.

Rotation of the shaft 12 in the opposite direction can ultimately cause the deformable arms 52, 54 to retract toward the longitudinal axis of the orthopaedic device 10, 36, thereby facilitating the removal of the device, if required.

To assemble the orthopaedic device 10, the second threadable body 30 is engaged with the second threaded portion 18b of the shaft 12. The shaft 12 is then inserted coaxially into the expandable sections 20, 22, and the first threadable body 28 is engaged with the first threaded portion 18a the shaft 12. Once assembled, each of the threadably mountable bodies 28, 30 is located on one of the threaded portions 18a, 18b of the shaft 12. Each of the mountable bodies 28, 30 can be attached to each respective end 44, 50 of the expandable sections 20, 22 by an adherent means, such as an adhesive.

In use, the shaft 12 is rotated using a hex key or similar torque delivery device, inserted into the hexagonal socket is provided at the terminal ends of the shaft 12. Rotation of the shaft 12 causes the first threadable body 28 to advance along the first threaded portion 18a of the shaft 12 and the second threadable body 30 to advance, in the opposite direction, along the second threaded portion 18b of the shaft 12. This applies mechanical pressure to the first end 44 of the first expandable section 20 and fourth end 50 of the second expandable section 22, wherein the first end 44 of the first expandable section 20 and fourth end 50 of the second expandable section 22 are bought sequentially into closer proximity relative to one another, causing the deformable arms 52, 54 to deform at each of the points of weakness 56, 58, and to expand radially from the longitudinal axis of the orthopaedic device 10.

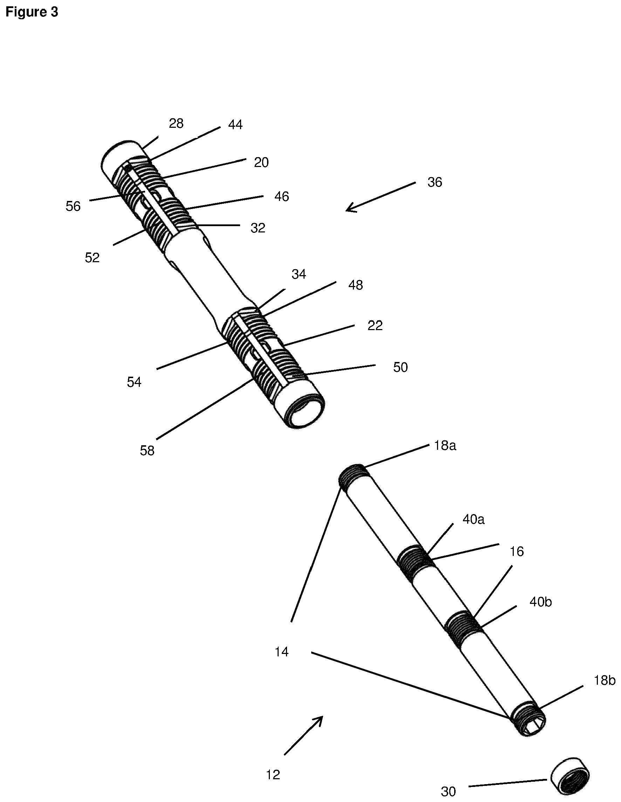

Referring to Figure 3 of the drawings, there is provided an orthopaedic device 36 according to a second embodiment of the present, comprising a connecting means, which in this embodiment is a shaft 12; two pairs of threaded portions wherein a first threaded pair 14 comprises a first threaded portion 18a and a second threaded portion 18b, a second threaded pair 16 comprises a third threaded portion 40a and a fourth threaded portion 40b, wherein the threads of the first threaded pair comprising the first threaded portion 18a and second threaded portion 18b are of reverse orientation to each other and the threads of the second threaded pair comprising the third threaded portion 40a and the fourth threaded portion 40b are of reverse orientation to each other, additionally, the first threaded portion 18a and third threaded portion 40a are of reverse orientation to each other and the second threaded portion 18b and the fourth threaded portion 40b are of reverse orientation to each other; two expandable sections - a first expandable section 20 and a second expandable section 22 - the first 20 and second 22 expandable sections displaceable between a contracted position and an expanded position; and expanding means, which in this embodiment is a first threadable body 28, a second threadable body 30, a third threadable body 32 and fourth threadable body 34, in operable association with the shaft 12 and the expandable sections 20, 22. **It** will be understood that Figure 1, Figure 4 and Figure 5 showing orthopaedic device 10 can also equally be orthopaedic device 36.

The shaft 12 allows reciprocal movement of the threadably mountable bodies 28, 30, 32, 34 relative to the shaft 12. The threaded portions 18a, 18b, 40a, 40b on the shaft 12 are each helically threaded portions. The threads of the first helically threaded portion 18a are in reverse orientation to the threads of the second helically threaded portion 18b. The threads of the third helically threaded portion 40a are in reverse orientation to the threads of the fourth helically threaded portion 40b. The threads of the first helically threaded portion 18a are in reverse orientation to the threads of the third helically threaded portion 40a. The threads of the second helically threaded portion 18b are in reverse orientation to the threads of the fourth helically threaded portion 40b. The two helical screw threaded portions 18a, 18b are provided adjacent each terminal end of the shaft 12. The two helical screw threaded portions 40a, 40b are each provided in between the first helical screw threaded 18a and second helical screw threaded 18b, wherein helical screw threaded portion 40a is positioned between helical screw threaded portion 18a and helical screw threaded portion 40b; and helical screw threaded portion 40b is positioned between helical screw threaded portion 40a and helical screw threaded portion18b.

The shaft 12 is generally cylindrical in shape and each terminal end of the shaft 12 comprises a recess to actuate the shaft 12. One end of the shaft 12 is dimensioned and arranged to provide means for actuating the shaft 12, for example by providing means for delivering torque to the shaft. The terminal ends of the shaft 12 can be dimensioned and arranged to receive a torque delivery device such as a screwdriver, hex key, or similar device. A hexagonal socket (not shown) can be provided at the terminal ends of the shaft 12, and can be shaped and adapted to receive a hex key or similar torque delivery device.

In an embodiment, the threaded portions 18a, 18b, 40a, 40b are equally spaced apart on the shaft 12. In use, the orthopaedic device 36 comprising the shaft 12 crosses the fracture site so that the first threaded pair 14 comprising the first threaded portion 18a and third threaded portion are on one side of the fracture site and the second threaded pair 16 comprising the fourth threaded portion 40b and the second threaded portion 18b is on the other side of the fracture site.

The threadably mountable bodies 28, 30, 32, 34 are annular in shape having an internal threaded surface and open at both ends. The inner shape of the threadably mountable bodies 28, 30, 32, 34 are shaped and dimensioned to engage with the threaded shaft of an orthopaedic device.

A helical screw thread can be provided on the inner surface of each threadably mountable body 28, 30, 32, 34. The internal diameter of each threadably mountable body 28, 30, 32, 34 is generally of similar length to the external diameter of the shaft 12, whereby the screw thread of each threadably mountable body 28, 30, 32, 34 can engage with the respective screw threaded portions 18a, 18b, 40a, 40b of the shaft 12.

Each expandable section 20, 22 comprise a generally cylindrically shaped tube, which is open at both ends. The first expandable section 20 and the second expandable section 22 are located coaxially. The first expandable section 20 comprises deformable arms 52, the second expandable section 22 also comprises deformable arms 54, such that the expandable sections 20, 22 comprises a plurality of arms 52, 54 that extend from the longitudinal axis of the orthopaedic devices 10, 36 under mechanical pressure. A point of weakness 56 is provided at the first end 44 and second end 46 of the first expandable section of the deformable arm 52, and the centre point of the length of the deformable arm 52. A point of weakness 58 is also provided at the third end 48 and fourth end 50 of the second expandable section of the deformable arm 54, and the centre point of the length of the deformable arm 54. Each deformable arm 52, 54 have at least one point of folding 56, 58 along each deformable arm 52, 54. Each of the points of folding 56, 58 comprises a point of weakness, a hinge mechanism, or any such mechanism that will facilitate the folding of the deformable arm at a desired location.

The first 20 and second 22 expandable sections can be reversibly expandable. The first 20 and second 22 expandable sections are collapsible along their longitudinal axis, such that the first 20 and second 22 expandable sections are radially inwardly collapsible.

Rotation of the shaft 12 in the opposite direction can ultimately cause the deformable arms 52, 54 to retract toward the longitudinal axis of the orthopaedic device 10, 36, thereby facilitating the removal of the device, if required.

To assemble the orthopaedic device 36, the second threadable body 30 is engaged with the second threaded portion 18b of the shaft 12, the fourth threadable body 34 is engaged with the fourth threaded portion 40b of the shaft 12 and the third threadable body 32 is engaged with the third threaded portion 40a of the shaft 12. The shaft 12 is then inserted coaxially into the expandable sections 20, 22, and the first threadable body 28 is engaged with the first threaded portion 18a the shaft 12. Once assembled, each of the threadably mountable bodies 28, 30, 32, 34 is located on one of the threaded portions 18a, 18b, 40a, 40b of the shaft 12. Each of the mountable bodies 28, 30, 32, 34 can be attached to each respective end 44, 46, 48, 50 of the expandable sections 20, 22 by an adherent means, such as an adhesive.

In an alternative assembly of the orthopaedic device 36, the second threadable body 30 is engaged with the second threaded portion 18b of the shaft 12. The shaft 12 is then inserted coaxially into the second expandable section 22 and the fourth threadable body 34 is engaged with the fourth threaded portion 40b the shaft 12. Separately, the third threadable body 32 is engaged with the third threaded portion 40a of the shaft 12. The shaft 12 is then inserted coaxially into the second first section 20 and the first threadable body 28 is engaged with the first threaded portion 18a the shaft 12. Once assembled, each of the threadably mountable bodies 28, 30, 32, 34 is located on one of the threaded portions 18a, 18b, 40a, 40b of the shaft 12. Each of the mountable bodies 28, 30, 32, 34 can be attached to each respective end 44, 46, 48, 50 of the expandable sections 20, 22 by an adherent means, such as an adhesive.

In use, the shaft 12 is rotated using a hex key or similar torque delivery device, inserted into the hexagonal socket is provided at the terminal ends of the shaft 12. Rotation of the shaft 12 causes the first threadable body 28 to advance along the first threaded portion 18a of the shaft 12; and the second threadable body 30 to advance, in the opposite direction, along the second threaded portion 18b of the shaft 12, the third threadable body 32 body to advance along the third threaded portion 40a of the shaft 12 and the fourth threadable body 34 to advance, in the opposite direction, along the second threaded portion 40b of the shaft 12. Additionally and simultaneously, rotation of the shaft 12 causes the first threadable body 28 to advance along the first threaded portion 18a of the shaft 12 and the third threadable body 32 to advance, in the opposite direction, along the third threaded portion 40a of the shaft 12, the second threadable body 30 body to advance along the second threaded portion 18b of the shaft 12 and the fourth threadable body 34 to advance, in the opposite direction, along the second threaded portion 40b of the shaft 12. This applies mechanical pressure to the first end 44 and second end 46 of the first expandable section 20, and the third end 48 and fourth end 50 of the second expandable section 22, wherein the first end 44 and second end 46 of the first expandable section 20, and the third end 48 and fourth end 50 of the second expandable section 22 are bought sequentially into closer proximity relative to one another, causing the deformable arms 52, 54 to deform at each of the points of weakness 56, 58, and to expand radially from the longitudinal axis of the orthopaedic device 36.

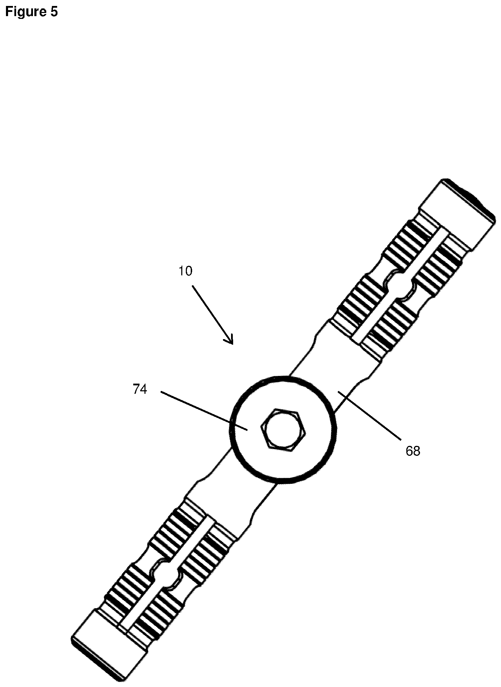

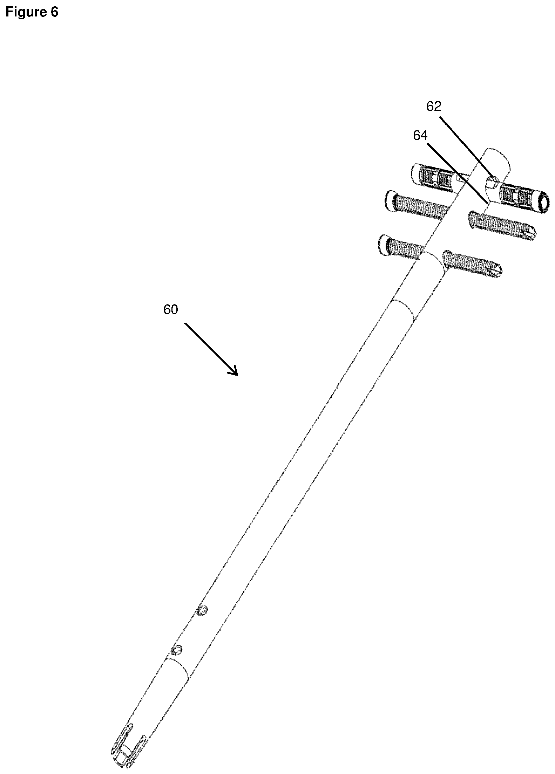

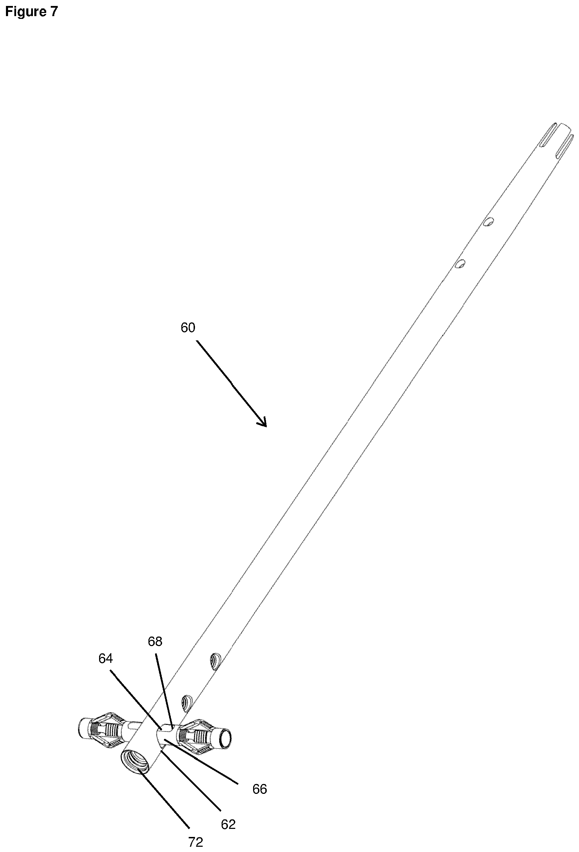

Referring to Figure 5, Figure 6 and Figure 7, there is provided an orthopaedic system comprising an orthopaedic device 10, 36 according to a first aspect of the present invention; and an orthopaedic implant, which in this embodiment, is an intramedullary nail 60.

The intramedullary nail 60 comprises an aperture comprising a first part 62 and a second part 64, wherein the first part 62 is circular and can receive the curved section 66 of the orthopaedic device 10, 36, and the second part 64 is non-circular and can receive the planar section 68 of the orthopaedic device 10, 36.

In use, the orthopaedic device 10, 36 according to a first aspect of the present invention, is inserted into the first part 62 of the aperture in the intramedullary nail 60. A set-screw 70 is threadably mountable to the longitudinal aperture 72 of the intramedullary nail 60. The outer surface 74 of the set-screw 70 is dimensioned and arranged to provide means for actuating the set screw 70, for example by providing means for delivering torque to the set-screw 70. The outer surface 74 of the set-screw 70 can be dimensioned and arranged to receive a torque delivery device such as a screwdriver, hex key, or similar device. The set-screw 70, by the application of torque, is driven through the longitudinal aperture 72 of the intramedullary nail 60 in the direction of the longitudinal axis of intramedullary nail 60 the towards the first part 62 of the aperture in the intramedullary nail 60 until the set-screw 70 engages with the curved section 62 of the orthopaedic device 10, 36. The set-screw 70, by the application of further torque, displaces the orthopaedic device 10, 36 from the first part 62 of the aperture to the second part 64 of the aperture to secure the orthopaedic device 10, 36 therein, thus preventing further rotation of the orthopaedic device 10, 36 in the intramedullary nail 60 and assuring that the expandable sections 20, 22 of the orthopaedic device 10, 36 do not collapse under external pressure from the surrounding bone, with which it is in contact.

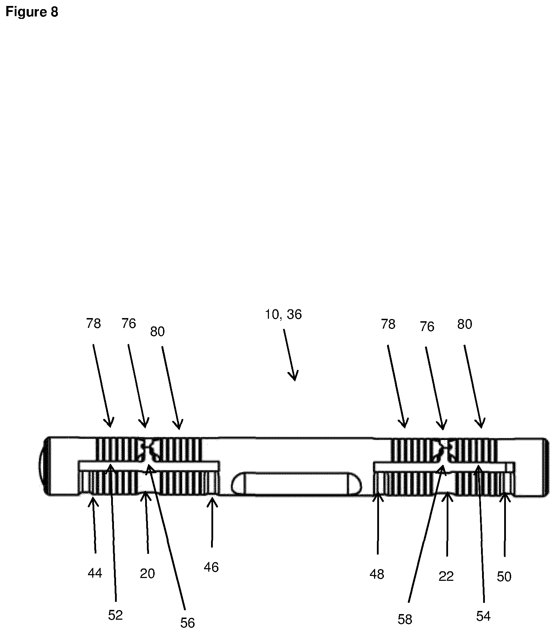

Alternatively, there is provided an orthopaedic device according to Figure 8, wherein the orthopaedic device further comprises limiting means.

In use, the shaft 12 is rotated using a hex key or similar torque delivery device, inserted into the hexagonal socket is provided at the terminal ends of the shaft 12. Rotation of the shaft 12 causes the first threadable body 28 to advance along the first threaded portion 18a of the shaft 12 and the second threadable body 30 to advance, in the opposite direction, along the second threaded portion 18b of the shaft 12. This applies mechanical pressure to the first end 44 of the first expandable section 20 and fourth end 50 of the second expandable section 22, wherein the first end 44 of the first expandable section 20 and fourth end 50 of the second expandable section 22 are bought sequentially into closer proximity relative to one another causing the deformable arms 52, 54 to deform at each of the points of weakness 56, 58, and to expand radially from the longitudinal axis of the orthopaedic device 10 and also closes the space 76 between the non-contiguously arranged limiting means, which in this instance is a first strut 78 and a second strut 80, until the first strut 78 and a second strut 80 meet, thereby limiting displacement between the contracted position and the expanded position.

Alternatively, in use, the shaft 12 is rotated using a hex key or similar torque delivery device, inserted into the hexagonal socket is provided at the terminal ends of the shaft 12. Rotation of the shaft 12 causes the first threadable body 28 to advance along the first threaded portion 18a of the shaft 12; and the second threadable body 30 to advance, in the opposite direction, along the second threaded portion 18b of the shaft 12, the third threadable body 32 body to advance along the third threaded portion 40a of the shaft 12 and the fourth threadable body 34 to advance, in the opposite direction, along the second threaded portion 40b of the shaft 12. Additionally and simultaneously, rotation of the shaft 12 causes the first threadable body 28 to advance along the first threaded portion 18a of the shaft 12 and the third threadable body 32 to advance, in the opposite direction, along the third threaded portion 40a of the shaft 12, the second threadable body 30 body to advance along the second threaded portion 18b of the shaft 12 and the fourth threadable body 34 to advance, in the opposite direction, along the second threaded portion 40b of the shaft 12. This applies mechanical pressure to the first end 44 and second end 46 of the first expandable section 20, and the third end 48 and fourth end 50 of the second expandable section 22, wherein the first end 44 and second end 46 of the first expandable section 20, and the third end 48 and fourth end 50 of the second expandable section 22 are bought sequentially into closer proximity relative to one another, causing the deformable arms 52, 54 to deform at each of the points of weakness 56, 58, and to expand radially from the longitudinal axis of the orthopaedic device 10 and also closes the space 76 between the non-contiguously arranged limiting means, which in this instance is a first strut 78 and a second strut 80, until the first strut 78 and a second strut 80 meet, thereby displacement between the contracted position and the expanded position.

In some embodiments, the limiting means is a stop, which radially expands from the shaft 12 thereby preventing the first end of the first expandable section 44 and/or the fourth end of the second expandable section 55 from moving sequentially into closer proximity relative to one another, thereby limiting the expansion of the at least one expandable section 20, 22.

The advantage of limiting expansion ensures uniform and reproducible expansion of the respective expandable sections and prevents preferential over-expansion of one expandable section versus the other expandable section. For example, in some embodiments, the first expandable section 20 may preferentially over-expand in comparison to the second expandable section 22 and limiting displacement of the first expandable 20 section may be preferable to ensure uniform expansion and ensure weight bearing post-operatively is consistent. In some embodiments, the second expandable section 22 may preferentially over-expand in comparison to the first expandable section 20 and limiting displacement of the second expandable section 22 may be preferable to ensure uniform expansion and ensure weight bearing post-operatively is consistent. In some embodiments, it may be preferable to limit displacement of both the first expandable section 20 and the second expandable section 22 to ensure uniform expansion and ensure weight-bearing load is consistent.

In some embodiments, one expandable section, in this instance, the first expandable section 20 comprises the limiting means. Rotation of the shaft 12 causes the first threadable body 28 to advance along the first threaded portion 18a of the shaft 12 and the second threadable body 30 to advance, in the opposite direction, along the second threaded portion 18b of the shaft 12. This applies mechanical pressure to the first end 44 of the first expandable section 20 and fourth end 50 of the second expandable section 22, wherein the first end 44 of the first expandable section 20 and fourth end 50 of the second expandable section 22 are bought sequentially into closer proximity relative to one another causing the deformable arms 52, 54 to deform at each of the points of weakness 56, 58, and to expand radially from the longitudinal axis of the orthopaedic device 10. The limiting means on the first expandable section 20 limits displacement of the first expandable section 20 between the contracted position and the expanded position by preventing the first end of the first expandable section 44 from moving sequentially into closer proximity to the fourth end of the second expandable section 55, thereby limiting the displacement of the at least one expandable section 20, 22, whilst allowing the second expandable section 22 to preferentially expand and thereby causing uniform expansion.

Alternatively, the second expandable section 22 comprises the limiting means. Rotation of the shaft 12 causes the first threadable body 28 to advance along the first threaded portion 18a of the shaft 12 and the second threadable body 30 to advance, in the opposite direction, along the second threaded portion 18b of the shaft 12. This applies mechanical pressure to the first end 44 of the first expandable section 20 and fourth end 50 of the second expandable section 22, wherein the first end 44 of the first expandable section 20 and fourth end 50 of the second expandable section 22 are bought sequentially into closer proximity relative to one another causing the deformable arms 52, 54 to deform at each of the points of weakness 56, 58, and to expand radially from the longitudinal axis of the orthopaedic device 10. The limiting means on the second expandable section 22 limits expansion of the second expandable section 22 between the contracted position by preventing the fourth end of the second expandable section 55 from moving sequentially into closer proximity relative to the first end of the first expandable section 44, thereby limiting the displacement of the at least one expandable section 20, 22, whilst allowing the second expandable section 22 to preferentially expand and thereby causing uniform expansion.

Further alternatively, both the first expandable section 20 and the second expandable section 22 comprise the limiting means. Rotation of the shaft 12 causes the first threadable body 28 to advance along the first threaded portion 18a of the shaft 12 and the second threadable body 30 to advance, in the opposite direction, along the second threaded portion 18b of the shaft 12. This applies mechanical pressure to the first end 44 of the first expandable section 20 and fourth end 50 of the second expandable section 22, wherein the first end 44 of the first expandable section 20 and fourth end 50 of the second expandable section 22 are bought sequentially into closer proximity relative to one another causing the deformable arms 52, 54 to deform at each of the points of weakness 56, 58, and to expand radially from the longitudinal axis of the orthopaedic device 10. The limiting means on the first expandable section 20 and the second expandable section 22 limits displacement of the first expandable section 20 and the second expandable section 22 between the contracted position by preventing the first end of the first expandable section 44 and fourth end of the second expandable section 55 from moving sequentially into closer proximity relative to each other, thereby limiting the displacement of both expandable sections 20, 22.

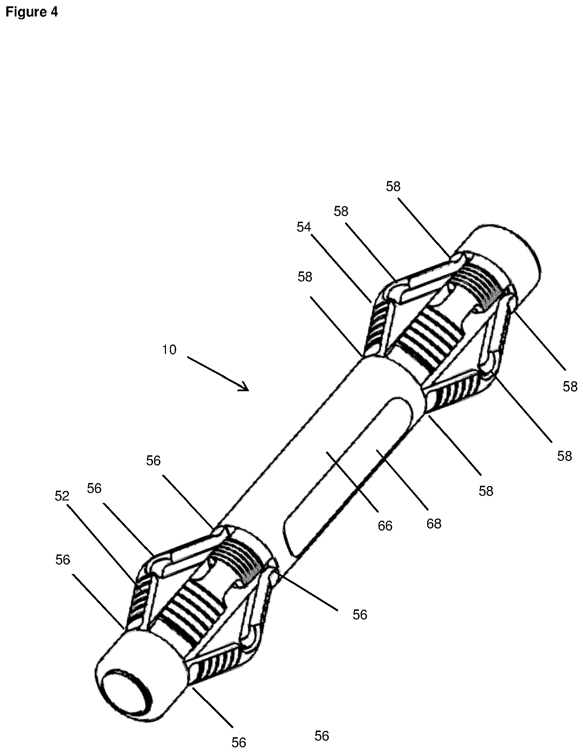

**It** will be understood that Figure 4 and Figure 5 showing orthopaedic device 10 can also equally be orthopaedic device 36.

In an embodiment, the aperture in the intramedullary nail is circular and orthopaedic device is circular in shape.

In a further embodiment, the aperture in the intramedullary nail is substantially non-circular and orthopaedic device is substantially non-circular in shape.

In a further embodiment, the aperture in the intramedullary nail circular and orthopaedic device is substantially non-circular in shape.

In a further embodiment, the aperture in the intramedullary nail is substantially non-circular and orthopaedic device is circular in shape.

The orthopaedic devices of the present invention 10, 36 are formed of a material that is suitable for sterilisation, such as an autoclavable material, so as to be provided in a sterile packaged state for use. The material can be surgical stainless steel, but it will be seen that any material that is suitable for sterilisation and can impart the required mechanical strength may be used.

There is also provided a method for fixation of bones comprising the steps of reducing the fracture by utility of orthopaedic devices of the present invention 10, 36. A pathway across the fractured bone is prepared using a surgical drill, or similar device. When the pathway has been prepared, the orthopaedic device 10, 36, once assembled and with the expandable sections 20, 22 in a contracted position, the orthopaedic device 10, 36 is placed into the pathway. The deformable arms 52, 54 of the expandable sections 20, 22 are radially expanded using, for example, a screwdriver inserted into, for example, a hex socket in the terminal end of the connecting means 12, for example the shaft 12. Optionally, the method comprises providing an intramedullary nail 60 used in conjunction with the orthopaedic device 10, 36.

In an embodiment for the fixation of bones, the threaded portions 18a, 18b are spaced apart at the terminal ends of the shaft 12. In use, the orthopaedic device 10 comprising the shaft 12 crosses the fracture site so that the first threaded portion 18a is one side of the fracture site and the second threaded portion 18b is on the other side of the fracture site.

In an embodiment for the fixation of bones, the threaded portions 18a, 18b, 40a, 40b are equally spaced apart on the shaft 12. In use, the orthopaedic device 36 comprising the shaft 12 crosses the fracture site so that the first threaded pair 14 comprising the first threaded portion 18a and third threaded portion are on one side of the fracture site and the second threaded pair 16 comprising the fourth threaded portion 40b and the second threaded portion 18b is on the other side of the fracture site.

The present invention finds utility as an apparatus for fixation of bones such as fractures of the distal femur or proximal tibia.

For distal femoral fractures, the patient is anaesthetised, and positioned supine on the operating table with the affected leg flexed to about 45 degrees. A medial parapatellar incision is made, and access is made to the intercondylar notch of the femur from within the knee joint. The starting point for the nailing is confirmed by anteroposterior (AP) and lateral fluoroscopy. A guidewire is passed from the centre of the intercondylar notch, in line with the vertical axis of the femur, aiming for the mid-point of the femur along the vertical axis in both planes. The guidewire is passed across the fracture site and into the proximal fragment.

The channel for the orthopaedic implant / intramedullary nail is prepared by reaming the intramedullary cavity with appropriately sized reamers. The correct length and diameter of the orthopaedic implant / intramedullary nail are selected from x-ray templating, or using a ruler overlaying the femur and using fluoroscopy. The appropriately sized nail is mounted on the alignment jig. The orthopaedic implant / intramedullary nail is inserted over the guidewire, across the fracture site and into the proximal femur. The distal part of the orthopaedic implant / intramedullary nail should be sunk 3-5 mm within the bone at the intercondylar notch.

The correct orientation and trajectory of the orthopaedic device is identified with a guidewire trough the appropriate aperture on the alignment jig and via a stab incision over the lateral distal femur. The correct length of orthopaedic device is measured via the guidewire and sleeve, noting the distance on either side of the nail. The orthopaedic device should be similar or slightly shorter in length than the transverse width of the distal femur.

The channel for the orthopaedic device is drilled over the guidewire, both of which are then removed. The orthopaedic device is inserted via the sleeve and alignment jig and advanced into position.

The rotation of the orthopaedic device is noted, so the flat aspects on the shaft are facing along the longitudinal axis of the nail.

The set-screw is now advanced via a screwdriver up from the distal end of the nail, to engage with the orthopaedic device. Further advancement of the set-screw retracts the nail downwards towards the knee, until the orthopaedic device is seated within the narrower keyed part of the nail aperture. This now secures the orthopaedic device from spinning when deployed. The orthopaedic device is deployed via clockwise rotation of the screwdriver. Expansion may continue until a stop is felt or the surgeon is satisfied with the position on fluoroscopy. The screwdriver is removed, and further locking screws may be inserted into the nail, completing the overall construct.

The alignment jig is removed, and an end cap is inserted onto the distal part of the nail to prevent bone ingrowth and to reduce blood and bone marrow from entering the knee joint.

Final construct positions are checked on fluoroscopy, wounds are closed and dressed.

## Claims

1. An orthopaedic device (10) comprising:
(a) connecting means comprising a shaft (12) comprising at least a first threaded portion (18a) anc a second threaded portion, (18b) wherein the threads of the first threaded portion are of reverse orientation to the threads of the second threaded portion;
(b) a first expandable section (20) comprising a first end (44) and a second end (46), and a second expandable section (22) comprising a third end (48) and a fourth end (50), each expandable section displaceable between a contracted position and an expanded position; and
(c) expanding means in operable association with the connecting means and the expandable sections, the expanding means comprising a first body (28) threadably mountable to the first threaded portion of the connecting means and a second body (30) threadably mountable to the second threaded portion of the connecting means to allow for simultaneous movement of the first threaded body and the second threaded body in opposite directions relative to each other,
wherein rotation of the connecting means causes the expanding means to apply a force to the respective ends of the expandable sections, thereby causing displacement of the expandable sections between the contracted position and the expanded position.

2. The orthopaedic device according to claim 1 further comprising limiting means, wherein the limiting means limits displacement of at least one expandable section between the contracted position and the expanded position by preventing the respective ends of the expandable sections from moving sequentially into closer proximity relative to one another.

3. The orthopaedic device according to claim 1 or 2, wherein the first and second expandable sections are arranged contiguously and/or are reversibly expandable.

4. The orthopaedic device according to any one of claims 1-3, wherein the first threaded portion and the second threaded portion are located at or adjacent respective opposing ends of the shaft.

5. The orthopaedic device according to any one of claims 1-4, wherein rotation of the shaft causes the first threadable body to advance along the first threaded portion of the shaft and the second threadable body to advance, in the opposite direction, along the second threaded portion of the shaft, thereby applying mechanical pressure to the first end of the first expandable section and fourth end of the second expandable section, wherein the first end of the first expandable section and fourth end of the second expandable section are brought sequentially into closer proximity relative to one another thereby displacing the first and second expandable sections between the contracted position and the expanded position.

6. The orthopaedic device according to any one of claims 1-5, wherein the shaft comprises two pairs of threaded portions, a first pair (14) comprising the first threaded portion and seconc threaded portion; and a second pair (16) comprising a third threaded portion and a fourth threaded portion.

7. The orthopaedic device according to claim 6, wherein the threads of the first threaded portion are in reverse orientation to the threads of the second threaded portion, the threads of the third threaded portion are in reverse orientation to the threads of the fourth threaded portion, the threads of the first threaded portion are in reverse orientation to the threads of the third threaded portion and the threads of the second threaded portion are in reverse orientation to the threads of the fourth threaded portion.

8. An orthopaedic system comprising:
(a) the orthopaedic device according any one of claim 1-7; and
(b) an orthopaedic implant.

9. The orthopaedic system according to claim 8, wherein the orthopaedic device has a substantially non-circular cross-section comprising at least one linear section and at least one curved section (66).

10. The orthopaedic system according to claim 8 or 9, wherein an outer surface of the orthopaedic device comprises at least one planar section (68) and at least one curved section (66).

11. The orthopaedic system according to claim 10, wherein the orthopaedic device has a substantially non-circular cross-section comprising at least one linear section and at least one curved section, wherein the least one linear cross-section section corresponds to the at least one planar section on the outer surface of the orthopaedic device, and the at least one curved cross-section corresponds to the at least one curved section on the outer surface of the orthopaedic device.

12. The orthopaedic system according to any one of claims 8-11, wherein the orthopaedic implant further comprises at least one transverse aperture which bisects the longitudinal axis relative to the orthopaedic implant.

13. The orthopaedic system according to claim 12, wherein the transverse aperture comprises a first part and a second part, wherein the first part is circular to receive the curved outer section of the orthopaedic device, and the second part is non-circular to receive the flat outer section of the orthopaedic device.

14. The orthopaedic system according to any one of claims 8-13, wherein the orthopaedic implant further comprises a longitudinal aperture, wherein the longitudinal aperture (72) is coaxia with the longitudinal axis, and the longitudinal aperture comprises an inner threaded surface which is in mechanical communication with the transverse aperture.

15. The orthopaedic system according to any one of claims 8-14, further comprising locking means.

## Patentansprüche

1. Orthopädische Vorrichtung (10), umfassend:
(a) Verbindungsmittel, umfassend eine Welle (12), die mindestens einen ersten Gewindeabschnitt (18a) und einen zweiten Gewindeabschnitt (18b) umfasst, wobei die Gewinde des ersten Gewindeabschnitts entgegengesetzt zu den Gewinden des zweiten Gewindeabschnitts ausgerichtet sind;
(b) einen ersten ausdehnbaren Abschnitt (20), der ein erstes Ende (44) und ein zweites Ende (46) umfasst, und einen zweiten ausdehnbaren Abschnitt (22), der ein drittes Ende (48) und ein viertes Ende (50) umfasst, wobei jeder ausdehnbare Abschnitt zwischen einer zusammengezogenen Position und einer ausgezogenen Position verschiebbar ist; und
(c) Ausdehnungsmittel in funktionsfähiger Verbindung mit den Verbindungsmitteln und den ausdehnbaren Abschnitten, wobei die Ausdehnungsmittel einen ersten Körper (28), der mit dem ersten Gewindeabschnitt der Verbindungsmittel gewindeverbindbar ist, und einen zweiten Körper (30) umfassen, der mit dem zweiten Gewindeabschnitt der Verbindungsmittel gewindeverbindbar ist, um eine gleichzeitige Bewegung des ersten Gewindekörpers und des zweiten Gewindekörpers in entgegengesetzten Richtungen relativ zueinander zu erteilen,
wobei die Drehung der Verbindungsmittel bewirkt, dass die Ausdehnungsmittel eine Kraft auf die jeweiligen Enden der ausdehnbaren Abschnitte ausüben, wodurch eine Verschiebung der ausdehnbaren Abschnitte zwischen der zusammengezogenen Position und der ausgedehnten Position veranlasst wird.

2. Orthopädische Vorrichtung nach Anspruch 1, ferner umfassend Begrenzungsmittel, wobei die Begrenzungsmittel die Verschiebung mindestens eines ausdehnbaren Abschnitts zwischen der zusammengezogenen Position und der ausgedehnten Position begrenzen, indem sie verhindern, dass sich die jeweiligen Enden der ausdehnbaren Abschnitte nacheinander in eine genauere gegenseitige Nähe bewegen.

3. Orthopädische Vorrichtung nach Anspruch 1 oder 2, wobei der erste und der zweite ausdehnbare Abschnitt kontinuierlich angeordnet und/oder reversibel ausdehnbar sind.

4. Orthopädische Vorrichtung gemäß einem der Ansprüche 1 bis 3, wobei der erste Gewindeabschnitt und der zweite Gewindeabschnitt an oder benachbart zu den jeweiligen gegenüberliegenden Enden der Welle angeordnet sind.

5. Orthopädische Vorrichtung gemäß einem der Ansprüche 1 bis 4, wobei die Drehung der Welle bewirkt, dass sich der erste Gewindekörper entlang des ersten Gewindeabschnitts der Welle vorwärts bewegt und der zweite Gewindekörper sich in entgegengesetzter Richtung entlang des zweiten Gewindeabschnitts der Welle vorwärts bewegt, wodurch mechanischer Druck auf das erste Ende des ersten ausdehnbaren Abschnitts und das vierte Ende des zweiten ausdehnbaren Abschnitts aufgebracht wird, wobei das erste Ende des ersten ausdehnbaren Abschnitts und das vierte Ende des zweiten ausdehnbaren Abschnitts nacheinander in eine genauere gegenseitige Nähe gebracht werden, wodurch der erste und der zweite ausdehnbare Abschnitt zwischen der zusammengezogenen Position und der ausgedehnten Position verschoben werden.

6. Orthopädische Vorrichtung gemäß einem der Ansprüche 1 bis 5, wobei die Welle zwei Paare von Gewindeabschnitten umfasst, wobei ein erstes Paar (14) den ersten Gewindeabschnitt und den zweiten Gewindeabschnitt umfasst; und ein zweites Paar (16) einen dritten Gewindeabschnitt und einen vierten Gewindeabschnitt umfasst.

7. Orthopädische Vorrichtung nach Anspruch 6, wobei die Gewinde des ersten Gewindeabschnitts in entgegengesetzter Ausrichtung zu den Gewinden des zweiten Gewindeabschnitts stehen, die Gewinde des dritten Gewindeabschnitts in entgegengesetzter Ausrichtung zu den Gewinden des vierten Gewindeabschnitts stehen, die Gewinde des ersten Gewindeabschnitts in entgegengesetzter Ausrichtung zu den Gewinden des dritten Gewindeabschnitts stehen und die Gewinde des zweiten Gewindeabschnitts in entgegengesetzter Ausrichtung zu den Gewinden des vierten Gewindeabschnitts stehen.

8. Orthopädisches System, umfassend:
(a) die orthopädische Vorrichtung nach einem der Ansprüche 1 bis 7; und
(b) ein orthopädisches Implantat.

9. Orthopädisches System nach Anspruch 8, wobei die orthopädische Vorrichtung einen wesentlich nicht kreisförmigen Querabschnitt aufweist, der mindestens einen linearen Abschnitt und mindestens einen gerundeten Abschnitt (66) umfasst.

10. Orthopädisches System nach Anspruch 8 oder 9, wobei eine Außenfläche der orthopädischen Vorrichtung mindestens einen planaren Abschnitt (68) und mindestens einen gerundeten Abschnitt (66) umfasst.

11. Orthopädisches System nach Anspruch 10, wobei die orthopädische Vorrichtung einen wesentlich nicht kreisförmigen Querabschnitt aufweist, der mindestens einen linearen Abschnitt und mindestens einen gerundeten Abschnitt umfasst, wobei der mindestens eine lineare Querabschnitt dem mindestens einen planaren Abschnitt an der Außenfläche der orthopädischen Vorrichtung entspricht und der mindestens eine gerundete Querabschnitt dem mindestens einen gerundeten Abschnitt an der Außenfläche der orthopädischen Vorrichtung entspricht.

12. Orthopädisches System gemäß einem der Ansprüche 8 bis 11, wobei das orthopädische Implantat ferner mindestens eine Transversalöffnung umfasst, die die Längsachse relativ zum orthopädischen Implantat halbiert.

13. Orthopädisches System nach Anspruch 12, wobei die Transversalöffnung einen ersten Teil und einen zweiten Teil umfasst, wobei der erste Teil kreisförmig ist, um den gerundeten äußeren Abschnitt der orthopädischen Vorrichtung aufzunehmen, und der zweite Teil nicht kreisförmig ist, um den flachen äußeren Abschnitt der orthopädischen Vorrichtung aufzunehmen.

14. Orthopädisches System gemäß einem der Ansprüche 8 bis 13, wobei das orthopädische Implantat ferner eine Längsöffnung umfasst, wobei die Längsöffnung (72) koaxial zur Längsachse ist und die Längsöffnung eine innere Gewindefläche umfasst, die in mechanischer Kommunikation mit der Transversalöffnung steht.

15. Orthopädisches System gemäß einem der Ansprüche 8 bis 14, ferner umfassend Verriegelungsmittel.

## Revendications

1. Dispositif orthopédique (10) comprenant :
(a) des moyens de connexion comprenant un arbre (12) comprenant au moins une première partie filetée (18a) et une deuxième partie filetée (18b), dans lequel les filetages de la première partie filetée sont d'une orientation inverse des filetages de la deuxième partie filetée ;
(b) une première section extensible (20) comprenant une première extrémité (44) et une deuxième extrémité (46), et une seconde section extensible (22) comprenant une troisième extrémité (48) et une quatrième extrémité (50), chaque section extensible étant déplaçable entre une position contractée et une position étendue ; et
(c) des moyens d'extension en association fonctionnelle avec les moyens de connexion et les sections extensibles, les moyens d'extension comprenant un premier corps (28) pouvant être monté par filetage sur la première partie filetée des moyens de connexion et un second corps (30) pouvant être monté par filetage sur la deuxième partie filetée des moyens de connexion pour permettre le mouvement simultané du premier corps fileté et du second corps fileté dans des directions opposées l'un par rapport à l'autre,
dans lequel la rotation des moyens de connexion amène les moyens d'extension à appliquer une force aux extrémités respectives des sections extensibles, amenant ainsi les sections extensibles à se déplacer entre la position contractée et la position étendue.

2. Dispositif orthopédique selon la revendication 1, comprenant également des moyens de limitation, dans lequel les moyens de limitation limitent le déplacement d'au moins une section extensible entre la position contractée et la position étendue en empêchant les extrémités respectives des sections extensibles de se déplacer séquentiellement à une plus grande proximité l'une par rapport à l'autre.

3. Dispositif orthopédique selon la revendication 1 ou 2, dans lequel les première et seconde sections extensibles sont disposées de manière contiguë et/ou sont extensibles de manière réversible.

4. Dispositif orthopédique selon l'une quelconque des revendications 1 à 3, dans lequel la première partie filetée et la deuxième partie filetée sont situées au niveau ou à proximité des extrémités opposées respectives de l'arbre.

5. Dispositif orthopédique selon l'une quelconque des revendications 1 à 4, dans lequel la rotation de l'arbre amène le premier corps fileté à avancer le long de la première partie filetée de l'arbre et le second corps fileté à avancer, dans la direction opposée, le long de la deuxième partie filetée de l'arbre, appliquant ainsi une pression mécanique à la première extrémité de la première section extensible et à la quatrième extrémité de la seconde section extensible, dans lequel la première extrémité de la première section extensible et la quatrième extrémité de la seconde section extensible sont rapprochées séquentiellement l'une de l'autre, déplaçant ainsi les première et seconde sections extensibles entre la position contractée et la position étendue.

6. Dispositif orthopédique selon l'une quelconque des revendications 1 à 5, dans lequel l'arbre comprend deux paires de parties filetées, une première paire (14) comprenant la première partie filetée et la deuxième partie filetée ; et une seconde paire (16) comprenant une troisième partie filetée et une quatrième partie filetée.

7. Dispositif orthopédique selon la revendication 6, dans lequel les filetages de la première partie filetée sont d'une orientation inverse des filetages de la deuxième partie filetée, les filetages de la troisième partie filetée sont d'une orientation inverse des filetages de la quatrième partie filetée, les filetages de la première partie filetée sont d'une orientation inverse des filetages de la troisième partie filetée et les filetages de la deuxième partie filetée sont d'une orientation inverse des filetages de la quatrième partie filetée.

8. Système orthopédique comprenant :
(a) le dispositif orthopédique selon l'une quelconque des revendications 1 à 7 ; et
(b) un implant orthopédique.

9. Système orthopédique selon la revendication 8, dans lequel le dispositif orthopédique présente une section transversale sensiblement non circulaire comprenant au moins une section linéaire et au moins une section courbe (66).

10. Système orthopédique selon la revendication 8 ou 9, dans lequel une surface externe du dispositif orthopédique comprend au moins une section plane (68) et au moins une section courbe (66).

11. Système orthopédique selon la revendication 10, dans lequel le dispositif orthopédique présente une section transversale sensiblement non circulaire comprenant au moins une section linéaire et au moins une section courbe, dans lequel l'au moins une section transversale linéaire correspond à l'au moins une section plane sur la surface externe du dispositif orthopédique, et l'au moins une section transversale courbe correspond à l'au moins une section courbe sur la surface externe du dispositif orthopédique.

12. Système orthopédique selon l'une quelconque des revendications 8 à 11, dans lequel l'implant orthopédique comprend également au moins une ouverture transversale qui coupe l'axe longitudinal par rapport à l'implant orthopédique.

13. Système orthopédique selon la revendication 12, dans lequel l'ouverture transversale comprend une première partie et une seconde partie, dans lequel la première partie est circulaire pour recevoir la section externe courbe du dispositif orthopédique, et la seconde partie est non circulaire pour recevoir la section externe plate du dispositif orthopédique.

14. Système orthopédique selon l'une quelconque des revendications 8 à 13, dans lequel l'implant orthopédique comprend également une ouverture longitudinale, dans lequel l'ouverture longitudinale (72) est coaxiale à l'axe longitudinal, et l'ouverture longitudinale comprend une surface filetée interne qui est en communication mécanique avec l'ouverture transversale.

15. Système orthopédique selon l'une quelconque des revendications 8 à 14, comprenant également des moyens de verrouillage.
